# EUROPEAN PATENT SPECIFICATION

(11) **EP 3 220 754 B1**
(45) Date of publication and mention of the grant of the patent: **10.04.2019**
(21) Application number: 15790994.6
(22) Date of filing: 09.11.2015
(51) Int. Cl.: A23C 21/08, A23L 29/231, A23L 33/19, A61K 9/00, A61K 38/01, A61P 3/04, A23K 20/147, A23K 20/163

(54) **USE OF COMPLEXES OF WHEY PROTEIN MICELLES AND PECTIN FOR MANAGING BODY WEIGHT**
VERWENDUNG VON KOMPLEXEN VON MOLKEPROTEINMIZELLEN UND PEKTIN ZUR KONTROLLE DES KÖRPERGEWICHTS
UTILISATION DE COMPLEXES DE MICELLES DE PROTÉINES DE LACTOSÉRUM ET DE PECTINE POUR GESTION DE POIDS CORPOREL

(30) Priority: 19.11.2014 EP 14193832
(43) Date of publication of application: 27.09.2017
(73) Proprietor: Nestec S.A., 1800 Vevey (CH)
(72) Inventor: POUTEAU, Etienne, 75013 Paris (FR); ERKNER, Alfrun, CH-1010 Lausanne (CH); POPA NITA, Simina Florentina, CH-1110 Morges (CH); DONATO-CAPEL, Laurence, 1041 Poliez-le-Grand (CH)
(74) Representative: Couzens, Patrick John
(86) International application number: PCT/EP2015/076087
(87) International publication number: WO 2016/078954

(56) References cited:
- EP-A1- 2 074 891
- EP-A1- 2 583 565
- WO-A1-2004/022074
- WO-A1-2007/073188
- US-A1- 2011 250 310
- LAMBERS TIM T ET AL: "Fast and Slow Proteins: Modulation of the Gastric Behavior of Whey and Casein In Vitro", FOOD DIGESTION, SPRINGER-VERLAG, NEW YORK, vol. 4, no. 1, 23 March 2013 (2013-03-23), pages 1-6, XP035341593, ISSN: 1869-1978, DOI: 10.1007/S13228-012-0028-7 [retrieved on 2013-03-23]
- SOUZA FLAVIA N ET AL: "Production and characterization of microparticles containing pectin and whey proteins", FOOD RESEARCH INTERNATIONAL, vol. 49, no. 1, 27 July 2012 (2012-07-27), pages 560-566, XP028951902, ISSN: 0963-9969, DOI: 10.1016/J.FOODRES.2012.07.041

## Description

### Field of the Invention

The present invention relates to a composition comprising complexes of whey protein micelles and pectin for use in the treatment or prevention of obesity or being overweight. A further aspect of the invention relates to the non-therapeutic use of a composition comprising complexes of whey protein micelles and pectin. The invention is strictly defined by the claims.

### Background of the Invention

During the past decades, the prevalence of obesity has increased worldwide to epidemic proportion. Approximately 1 billion of people worldwide are overweight or obese, conditions that increase mortality, mobility and economical costs. Being overweight or obese is classically defined based on the percentage of body fat or, more recently, the body mass index or BMI. The BMI is defined as the ratio of weight in kg divided by the height in metres squared. Obesity develops when energy intake is greater than energy expenditure, the excess energy being stored mainly as fat in adipose tissue. Body weight loss and prevention of weight gain can be achieved by reducing energy intake or bioavailability, increasing energy expenditure and /or reducing storage as fat.

It has been known for many years that the ingestion of dietary proteins stimulates energy expenditure in the postprandial period immediately after meal ingestion. Certainly, on theoretical grounds, the energy cost of digesting, absorbing, and metabolizing proteins is greater than that of either carbohydrates or fat, and these theoretical values have been supported and confirmed for proteins and carbohydrates in human clinical trials [Tappy L. et al., 1993, Am J Clin Nutr, 57:912-916; Acheson K. et al., 1984, J Clin Invest, 74:1572-1580].

Proteins not only increase energy expenditure, but also decrease energy intake through mechanisms that influence appetite control [Halton TL. et al., 2004, J Am Coll Nutr, 23:373-385; Anderson GH. et al., 2004, J Nutr 134:974S-979S; Lejeune MP. et al., 2005, Br J Nutr, 93:281-289]. Thereby, the effect of hyper-aminoacidemia, especially of the branched chain amino acids and more importantly of leucine, is important to enhance energy expenditure that is partly a result of increased protein turnover. Results from many medium-term clinical trials have provided evidence that high-protein diets favour weight loss and reduce biomarkers of related metabolic diseases, at least over periods of several months to several years [Skov A.R. et al. 1999, Int J Obes 23:528-536; Brehm B.J. et al. 2003, J Clin Endocrinol Metab 88:1617-1623; Foster G.D. et al. 2003,N Engl J Med 348:2082-2090; Samaha F.F. et al., 2003, N Engl J Med 348:2074-2081; Due A. et al. 2004, Int J Obes Relat Metab Disord 28:1283-1290].

Further studies have shown that milk proteins are absorbed and digested at different rates, than for example animal and vegetable proteins, and thereby stimulate energy expenditure differently [Boirie Y et al., 1997, Proc Natl Acad Sci USA 94:14930-14935; Mikkelsen PB et al., 2000, Am J Clin Nutr 72:1135-1141]. Thus, different proteins appear to have a variety of acute and chronic metabolic effects, thereby affecting postprandial energy expenditure and satiety, and in the medium-term loss of weight, an increase of lean body mass and a decrease of body fat mass.

Acheson K et al. [2011, Am J Clin Nutr 93:525-534] investigated the thermic and metabolic responses and the satiating effects of 4 isocaloric test meals on 23 healthy men and women. Three of the meals provided 50% of the energy as whey, casein or soy proteins, respectively, and one meal was an iso-energetic high-carbohydrate meal as control. The results indicated that the energy expenditure as well as the thermic effect of the protein meals was higher than those of the carbohydrate rich meal. Further, among the protein rich meals, the whey protein meal showed the significantly strongest thermic effect and the largest energy expenditure effect on the tested subjects. Cumulative fat oxidation was also largest after the whey protein meal in comparison to the other 3 provided meals.

EP2583565 demonstrated that whey protein micelles may be used in the treatment or prevention of overweight and/or obesity in a subject.

WO 2004/022074 A1 and EP 2 074 891 A1, as well as Lambers et al., Food Digestion 2013, Vol. 4, (1), p. 1-6 describe compositions comprising microparticulated whey protein aggregates and pectin which are not based on complexes of micellar structures of whey protein and pectin and are characterised by cold gelling properties.

There is a persisting need to find better nutritional solutions for overweight subjects or subjects at risk of becoming overweight for better managing their body weight, e.g. through increasing satiety, postprandial energy expenditure, enhancing lean body mass and/or reducing body fat mass. The object of the present invention is to improve the state of the art and to provide an improved solution for the prevention or treatment of obesity and being overweight.

Any reference to prior art documents in this specification is not to be considered an admission that such prior art is widely known or forms part of the common general knowledge in the field. As used in this specification, the words "comprises", "comprising", and similar words, are not to be interpreted in an exclusive or exhaustive sense. In other words, they are intended to mean "including, but not limited to".

### Summary of the invention

Accordingly, the present invention provides in a first aspect a composition comprising complexes of whey protein micelles and pectin for use in the treatment or prevention of obesity, wherein the weight ratio of whey protein micelles to pectin in the composition is between 30:1 and 0.8:1, and the whey protein micelles are obtainable by adjusting the pH of a demineralized native whey protein aqueous solution to a value between 5.8 and 6.6 and subjecting the aqueous solution to a temperature between 80 and 98°C for a period of between 10 seconds and 2 hours.

In a second aspect, the invention relates to the non-therapeutic use of a composition comprising complexes of whey protein micelles and pectin to increase satiety and/or postprandial energy expenditure in a subject, wherein the weight ratio of whey protein micelles to pectin in the composition is between 30:1 and 0.8:1, and the whey protein micelles are obtainable by adjusting the pH of a demineralized native whey protein aqueous solution to a value between 5.8 and 6.6 and subjecting the aqueous solution to a temperature between 80 and 98°C for a period of between 10 seconds and 2 hours.

"Whey protein micelles" (WPM) are defined herein as described in EP1839492A1 and as further characterized in Schmitt C et al. [Soft Matter 6:4876-4884 (2010)], where they are referred to as whey protein microgels (WPM). Particularly, the "whey protein micelles" are the micelles comprised in the whey protein micelles concentrate obtainable by the process as disclosed in EP1839492A1. Therein, the process for the production of whey protein micelles concentrate comprises the steps of: a) adjusting the pH of a whey protein aqueous solution to a value between 3.0 and 8.0; b) subjecting the aqueous solution to a temperature between 80 and 98°C; and c) concentrating the dispersion obtained in step b). Thereby, the micelles produced have an extremely sharp size distribution, such that more than 80% of the micelles produced have a size smaller than 1 micron in diameter and preferably are between 100 nm and 900 nm in size. The "whey protein micelles" can be in liquid concentrate or in powder form. Importantly, the basic micelle structure of the whey proteins is conserved, in the concentrate, the powder and reconstituted from the powder for example in water. The "whey protein micelles" are physically stable in dispersion, as powder as well as during spray-drying or freeze-drying.

Being "overweight" is defined for an adult human as having a BMI between 25 and 30. BMI (body mass index) means the ratio of weight in kg divided by the height in metres, squared.

"Obesity" is a condition in which the natural energy reserve, stored in the fatty tissue of animals, in particular humans and other mammals, is increased to a point where it is associated with certain health conditions or increased mortality. "Obese" is defined for a human as having a BMI greater than 30.

It has now been surprisingly found by the inventors that consumption of a composition comprising complexes of whey protein micelles (WPM) and pectin by minipigs induces a more sustained amino acid absorption than consumption of an iso-caloric and iso-nitrogenous control composition with just whey protein micelles. The results of the pre-clinical study are presented in the Examples section. Hence, the inventors have found a composition which induces a sustained but high level of plasma amino acids in a subject. A high level of amino acids in the blood for a prolonged postprandial period of time is most favourable for maximally stimulating and increasing the postprandial energy expenditure, satiety and energy partitioning of the subject to improve body mass composition and control body weight. The resulting increase in thermogenesis will also enhance satiety. Circulating amino acids, and therefore the induced hyper-aminoacidemia can increase satiety via activation of the vagus nerve and act directly on the arcuate nucleus of the brain. Delayed amino acid appearance in the blood (prolonged hyper-aminoacedimia) indicates a delay in digestion of the proteins (including a delay in gastric emptying) and therefore a longer exposure to gastrointestinal hormones involved in satiety regulation to proteins, which means a longer signalling of satiety to the central nervous system.

"Hyper-aminoacidemia" is a high level of amino acids in the bloodstream, the amino acid pool, which can lead to an increase in both protein synthesis and protein breakdown through protein oxidation, with an overall positive nitrogen balance. Thereby, the positive nitrogen balance indicates more construction of lean tissue than destruction, leading overall to an increase in lean body mass and hence reduction of body fat mass.

Although not wishing to be bound by theory, the inventors think that whey protein micelles complexed with pectin induce a delayed gastric emptying or are more slowly digested than whey protein micelles alone. Thereby, complexes of whey protein micelles and pectin deliver the amino acids more slowly into the peripheral blood circulation.

### Brief Description of the Drawings

Figure 1: Variation of surface charge (ζ-potential) as a function of pH for WPM and pectin in solutions of concentration 0.1 wt % and at T = 25 °C.
Figure 2: Particle size distribution in WPM/pectin systems (at pH = 4) of protein concentration of 1 wt % and different pectin concentrations (weight ratios WPM:pectin between 1:1 and 10:1). Results are presented as scattered light intensity versus particle diameter in volume.
Figure 3: Particle size distribution in WPM/pectin systems (at pH = 4) of protein concentration of 1 wt % and different pectin concentrations (weight ratios WPM:pectin between 1:1 and 10:1). Results are presented as percentage of total volume versus particle diameter.
Figure 4: Leucine concentration in plasma (µM) versus time after meal for WPM (A) and WPM/pectin complexes (B).

### Detailed Description of the invention

The present invention relates to a composition comprising complexes of whey protein micelles and pectin for use in the treatment or prevention of obesity, wherein the weight ratio of whey protein micelles to pectin in the composition is between 30:1 and 0.8:1, for example between 10:1 and 1:1. The whey protein micelles in the composition of the invention are obtained by adjusting the pH of a demineralized native whey protein aqueous solution to a value between 5.8 and 6.6 and subjecting the aqueous solution to a temperature between 80 and 98°C for a period of between 10 seconds and 2 hours.

The invention may relate to the use of a composition comprising complexes of whey protein micelles and pectin for the manufacture of a medicament for use in the treatment or prevention of obesity, wherein the weight ratio of whey protein micelles to pectin in the composition is between 30:1 and 0.8:1. As the ratio of WPM to pectin increases above 30:1, the beneficial effect of the complexes becomes indistinguishable to WPM alone. For example, a minimum of 0.1% of the overall composition may be pectin on a dry weight basis, for further example a minimum of 2 % of the overall composition may be pectin on a dry weight basis. The pectin may be high methyl-esterified pectin. For ratios of WPM to pectin below 0.8:1, the compositions cannot provide sufficient protein to affect the plasma amino acids without becoming unacceptably viscous. The weight ratio of whey protein micelles to pectin in the complexes comprised within the composition of the invention may be between 30:1 and 0.8:1, for example between 10:1 and 1:1. The complexes may be used in the form of an aqueous dispersion, or they may be dried, for example to be used as a powder.

The composition for use according to the invention may be administered in combination with a meal. Most meals comprise proteins from a milk, plant and/or animal source and hence upon consumption lead to a postprandial aminoacidemia increase, i.e. an elevated concentration of amino acids in the plasma of the consumer. It is an advantage to combine the administration of WPM/pectin complexes with such a meal. Thereby, the postprandial plasma amino acid levels resulting from the proteins present in the meal are combined with the sustained postprandial amino acid levels resulting from the WPM/pectin complexes. Thereby, the overall resulting hyper-aminoacidemia is extended and prolonged in time. This in return is most favourable for maximally stimulating and increasing the postprandial energy expenditure, satiety and energy partitioning of the subject to improve body mass composition and control body weight.

The meal may comprise whey protein isolates, native or hydrolyzed milk proteins, free amino acids, or a combination thereof. As known from earlier studies, a whey protein meal exhibits a significantly stronger aminoacidemia effect on subjects than for example a plant protein meal. Therefore, advantageously, the WPM/pectin complexes are combined with a meal comprising whey proteins in the form of WPI or milk. Advantageously, the meal can be even further supplemented with free amino acids in combination with the whey or milk proteins to optimally induce a hyper-aminoacidemia upon consumption of said meal. The composition comprising WPM/pectin complexes may be provided as part of the meal in the form of a beverage, nutritional composition, bar, flakes or as pellets. Those forms of food product applications are ideal for incorporating WPM/pectin complexes in a sufficient quantity for providing the desired effect and still be acceptable by a consumer in view of the organoleptic aspect.

The composition comprising complexes of whey protein micelles and pectin for use according to the invention may be administered to a child or adult human being. Alternatively, they may be administered to a pet, for example a cat or a dog. Prevalence of obesity is mostly observed in adult humans. However, more and more children are affected as well and/or are already at risk of becoming overweight or obese later in life. Hence, advantageously, prevention and/or treatment of becoming overweight starts when young. Alternatively, and similarly as observed with humans, obesity is more and more widespread among animals, particularly with animals kept as pet animals. Hence, the invention also may pertain to cats and dogs.

The composition may be administered in a daily dose to provide between 0.1 g and 2.0 g dry weight of whey protein micelles and pectin per 1kg body weight, for example between 0.15 g and 1.5 g dry weight of whey protein micelles and pectin per 1 kg body weight. The composition may be administered in a daily dose to provide between 0.1 g and 2.0 g dry weight of complexes of whey protein micelles and pectin per 1 kg body weight, for example between 0.15 g and 1.5 g dry weight of complexes of whey protein micelles and pectin per 1 kg body weight. Those doses should assure a sufficient daily quantity for providing the desired effect to a subject in at least a mid-term period.

The composition may be in any convenient form, for example the composition may be in the form of a beverage, nutritional composition, bar, flakes or as pellets. The composition may be an oral nutritional support.

The composition may be a heat treated. An important method of controlling food hygiene risks is to heat treat edible compositions which may harbour food pathogens or spoilage organisms. Well-known examples of such heat treatments are pasteurization, for example heating an edible material to 72 °C for 15 seconds, and ultra-high temperature (UHT) treatment, for example heating an edible material to above 135 °C for at least 2 seconds.

The composition may be a heat treated liquid. Generally, the protein content that can be included in heat sterilized liquid compositions is greatly limited. Compositions with high contents of protein form thick gels on heating and so do not provide a convenient liquid format once heat treated. For example a native whey protein dispersion forms a gel in the presence of 0.1 M of sodium chloride at a protein concentration of only 4 wt.% after a heat treatment 85 °C for 15 min. The addition of pectin would be expected to make the problem of gelling worse. For example, the addition of pectin to whey protein has been found to decrease the protein gelling concentration or the gel time upon heat treatment [S.L. Turgeon et al., Food Hydrocolloids, 15, 583-591 (2001)]. The surprising finding that liquid compositions comprising WPM/pectin complexes may be heat treated and still remain liquid therefore allows an advantageous liquid composition to be provided. The composition for use according to the invention permits a large quantity of protein to be delivered in a relatively small volume without bad taste or texture. This is particularly advantageous for bariatric patients where consuming large volumes may be problematic. The heat treated liquid composition for use according to the invention may have a total content of whey protein micelles of at least 5 wt.%, for example at least 10 wt.%.

The liquid composition for use according to the invention may be a liquid meal replacement. The liquid meal replacement may be in a form suitable for enteral tube feeding. Advantageously such a meal replacement can for example be used in hospitals where patients, for example morbidly obese patients before or after bariatric surgery, require a controlled diet for recovery. A liquid meal replacement thereby is very convenient and provides the required amounts of proteins in a well-adapted formulation.

The total content of whey protein micelles in the composition for use according to the invention may be at least 5 wt.%, for example at least 10 wt.%. The total content of complexes of whey protein micelles and pectin in the composition for use according to the invention may be at least 5 wt.%, for example at least 10 wt.%.

As discussed above, it is of an advantage to combine the administration of WPM/pectin complexes with whey proteins in the form of WPI, milk and/or even free amino acids to optimally induce and extend a hyper-aminoacidemia upon consumption of such a meal. Preferably, the different protein components are combined together into one meal replacement product or kit of products. Thereby, the individual protein components can be optimally dosed for providing a best and prolonged hyper-aminoacidemia effect and at the same time optimized for a good, organoleptically best acceptable product application.

A further aspect of the present invention is the non-therapeutic use of a composition comprising complexes of whey protein micelles and pectin to increase satiety and/or postprandial energy expenditure in a subject, wherein the weight ratio of whey protein micelles to pectin in the composition is between 30:1 and 0.8:1, for example between 10:1 and 1:1. The protein micelles in the composition used according to the invention are obtained by adjusting the pH of a demineralized native whey protein aqueous solution to a value between 5.8 and 6.6 and subjecting the aqueous solution to a temperature between 80 and 98°C for a period of between 10 seconds and 2 hours.

It is an advantage of the present invention that compositions comprising complexes of whey protein micelles and pectin can also be administered to healthy subjects which may be at risk of becoming overweight. In fact, complexes of whey protein micelles and pectin, or compositions comprising them as disclosed herein provide healthy humans and animals with increased satiety and/or increased energy expenditure after consumption of said complexes of whey protein micelles and pectin. The effect is due to the herein disclosed sustained and prolonged hyper-aminoacidemia postprandial effect. Further, this effect is most favourable for improving body mass composition, such as enhancing lean body mass, and controlling body weight by decreasing for example body fat mass. The non-therapeutic use of the invention may be to enhance lean body mass and/or decrease body fat mass. Compositions comprising complexes of whey protein micelles and pectin may be used to help maintain a healthy body composition after weight loss. It is a legitimate desire for healthy subjects to wish to stay healthy and slim.

### Examples

### Example 1: Preparation of pectin-whey protein micelles complexes

Whey protein micelle powder (WPM) was produced by heat treatment at 85 °C/15min of a dispersion of whey protein isolate (Prolacta 90) at 4 %wt protein at pH 5.89, then concentration by microfiltration up to 22 %wt total solid and spray drying.

A pectin (high methyl-esterified pectin, Classic CU201, Herbstreith & Fox KG) stock solution of 5 wt.% was prepared in de-ionised water by stirring for 2-3 hours at 60 °C. To allow complete hydration of the chains, the solution was stirred overnight at 4 °C. A WPM stock solution of 15 %wt and pH 3.5 was prepared. Firstly, the powder was dispersed in a 135 mM HCI solution, overnight at 4 °C. The dispersion was then homogenized at 250 bars, 2 passes and at 50 bars, 1 pass. The final dry matter and subsequent protein concentration were verified using a HR73 Halogen Moisture Analyzer (Mettler Toledo) and the particle size was checked by dynamic light scattering (Zetasizer Nanoseries, Malvern, UK). Typical values were: hydrodynamic diameter Dh = 300 nm, polydispersity index pdl=0.15. Mixes of different protein concentrations (range 0.1 - 10 wt %) and WPM/pectin weight ratios (range 1:1 - 10:1) were obtained by blending the two solutions (and adding water if necessary). The mix was then homogenized at 500 bars for 2 passes at 25 °C. Final pH of the system was adjusted to pH 4.0 using 1M NaOH.

### Physicochemical characterization of the systems:

### - Surface charge

The surface charge corresponding to the electrophoretic mobility, the ζ-potential, of the particles was measured with a particle mobility distribution instrument (Zetasizer Nanoseries, Malvern, UK). A multipurpose titrator unit (MPT 2, Malvern) with 1M HCl and NaOH titrant solutions was used to vary the pH from 8 to 2 with an increment of 0.5 and a pH precision target of 0.3. A cell DTS1060C was used and the measurements were done at 25 °C. 15mL of 0.1 %wt solution was employed. The data processing was done automatically.

### - Particle size distribution

Particle size distribution was measured using multi-angle static light scattering with a Mastersizer S long bench (Malvern, UK). Refractive indices of 1.36 for the disperse phase and 1.33 for the continuous phase and a backscattering index of 0.1 (3JHD presentation) were used in the calculation. Residual values were always lower than 1.5. Taking into account the arbitrary choice of the refractive index of the disperse phase and the mathematical model used (which assumes particles are spherical), present measurements only provide a qualitative indication of the aggregation in the systems rather than a quantitative determination of particle sizes.

### Results

### I. Identification of pH conditions allowing formation of WPM/pectin electrostatic complexes

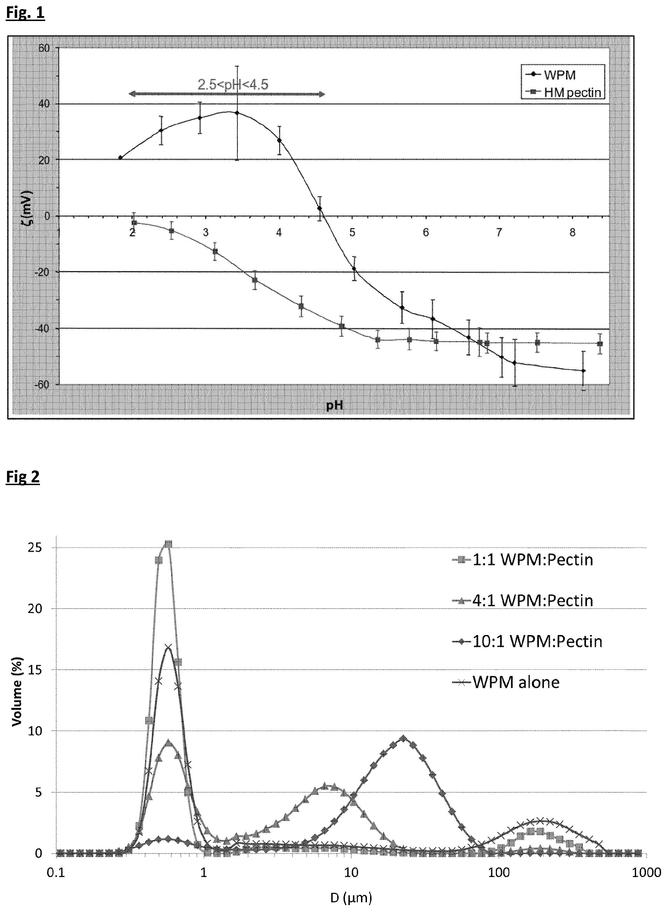

The surface charge (ζ-potential) of WPM and pectin as function of pH is illustrated in Figure 1. As pH increased from 2 to 8, the ζ-potential of pectin decreased from neutral to -45 mV. This variation can be related to the carboxyl groups on the pectin backbone, At low pH, the neutralization of these groups induced ζ-potential values close to zero. For WPM, the ζ-potential varied from 20 mV at pH 2 to 40 mV at pH 3.8 and decreased to -45 mV at pH 8 with electroneutrality measured at pH 4.6. The latter can be related to the isoelectric point of β-lactoglobulin, the main constitutive protein of the WPM.

These results showed that in the pH range 2.5-4.5 the two components carried opposite charges and thus, are susceptible to forming electrostatic complexes.

### II. Particle size distribution

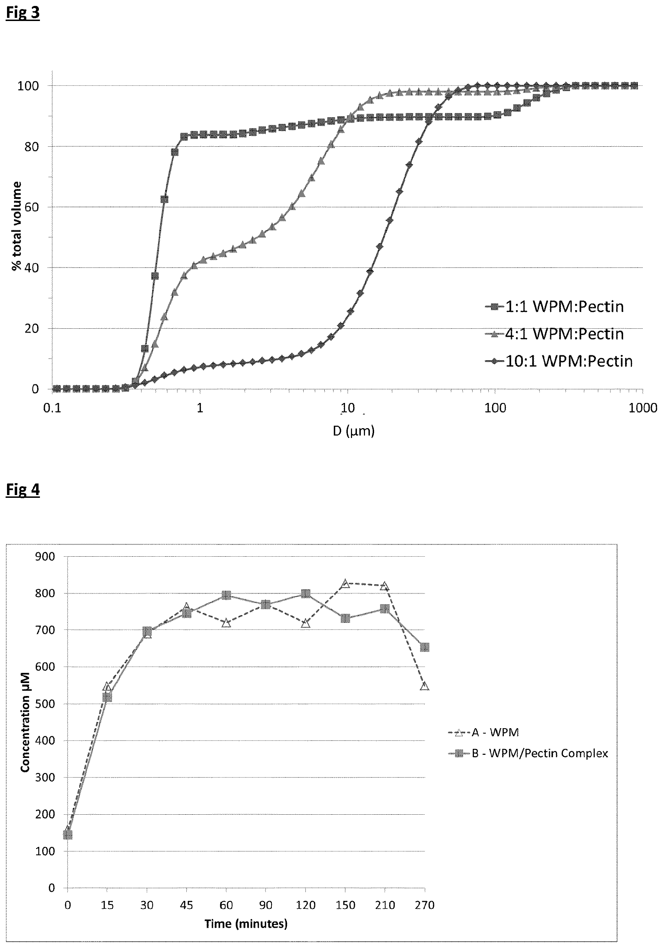

In order to evaluate the variations induced by pectin addition to WPM, particle size distribution was measured and Figures 2 and 3 present the results obtained for systems containing 1 wt % WPM and increasing amounts of pectin, from 0.1 wt % to 1 wt %, corresponding to WPM:pectin weight ratios of 10:1 to 1:1.

At low pectin concentration (0.1 wt %), the mean diameter of the particles was higher than 10 µm and less than 10 % of the total sample volume was represented by particles with diameters lower than 1 µm. As the pectin concentration increased up to 1 wt%, the mean diameter decreased below1 µm and more than 80 % of the total volume was represented by particles with diameters lower than 1 µm. At pectin concentration of 1 wt%, the average size of the particles was comparable to WPM alone. For high WPM:pectin ratios (i.e. low pectin concentrations), interactions between WPM and pectin are likely to occur due to charge effect and large aggregates are mainly formed. As pectin concentration increases, complexes comparable in size with WPM are formed probably due to compaction of pectin chains at the surface of the WPM.

The results show that an aqueous dispersion of pectin and whey protein micelles will form pectin-whey protein micelle complexes at pH conditions between 2.5 and 4.5.

### Example 2: Influence of complexes of whey protein micelles and pectin on amino acid appearance

The inventors monitored the postprandial response of plasma amino acid concentration in a randomized double-blinded crossover study in healthy minipigs. A wash-out period of at least 6 days was kept between two meals and during this time, minipigs were given regular diet.

The following iso-caloric and iso-nitrogenous meal replacements were compared.

| | |
|---|---|
| **A** | Whey protein micelles (WPM) + lipids + maltodextrin |
| **B** | WPM/pectin complexes + lipids + maltodextrin |

Both meals were approximately 300 ml and contained 30 g of whey protein, 11 g of lipid and 30 g of maltodextrin. Meal B also contained 1.5 g pectin (high methyl-esterified pectin, Classic CU201, Herbstreith & Fox KG). The calorific value and protein content were measured analytically and the size of each test meal slightly adjusted to ensure they were all iso-caloric and iso-nitrogenous. Meal A was at neutral pH and Meal B was at acidic pH.

Meal A: WPM powder was produced by heat treating a 4wt.% protein dispersion (pH 5.89) of WPI (Prolacta 90) at 85 °C for 15 minutes, then concentration by microfiltration up to 22 wt.% solids and spray drying. A 15 % t.s. solution (pH 7) of WPM was homogenised and mixed with a homogenised emulsion of 40 % oil in water stabilized by 4 % Citrem emulsifier. Maltodextrin (DE 21) was added, and the mixture underwent UHT treatment at 148 °C for 3 seconds before filling into sterile bottles.

Meal B: WPM powder was produced as for meal A. A 15 % t.s. solution (pH 4) of WPM was homogenised and mixed with pectin and maltodextrin at 60 °C for 1 hour to form WPM/pectin complexes. The mixture was then homogenized at 250 bar and mixed with a homogenised emulsion of 40 % oil in water stabilized by 4 % Citrem emulsifier. The pH was checked/adjusted to be pH 4. The mixture underwent UHT treatment at 148 °C for 3 seconds before filling into sterile bottles.

Blood samples were taken at 11 time points from 30 minutes before the meal to 270 minutes after, and the plasma leucine concentration determined. The results are plotted in Figure 4. The areas under the two curves are essentially the same, showing that the overall leucine delivered was the same. However, it can be seen that while the concentration of leucine starts to tail-off between 210 and 270 minutes for sample A (WPM), the leucine concentration remains higher for sample B (WPM/pectin) demonstrating a more sustained amino acid absorption. This study showed the advantage of compositions comprising WPM/pectin complexes for maintaining an elevated concentration of plasma amino acids in a subject.

## Claims

1. Composition comprising complexes of whey protein micelles and pectin for use in the treatment or prevention of obesity, wherein the weight ratio of whey protein micelles to pectin in the composition is between 30:1 and 0.8:1, and the whey protein micelles are obtainable by adjusting the pH of a demineralized native whey protein aqueous solution to a value between 5.8 and 6.6 and subjecting the aqueous solution to a temperature between 80 and 98°C for a period of between 10 seconds and 2 hours.

2. A composition for use according to claim 1 wherein the composition is administered in combination with a meal.

3. A composition for use according to claim 2 wherein the meal comprises whey protein isolates, native or hydrolysed milk proteins, free amino acids, or a combination thereof.

4. A composition for use according to any one of claims 1 to 3 wherein the composition is to be administered to a child or adult human being.

5. A composition for use according to any one of claims 1 to 4 wherein the composition is to be administered to a pet.

6. A composition for use according to any one of claims 1 to 5 wherein the composition is administered in a daily dose to provide between 0.1 g and 2.0 g dry weight of whey protein micelles and pectin per 1 kg body weight.

7. A composition for use according to any one of claims 1 to 6 wherein the composition is in the form of a beverage, nutritional composition, bar, flakes or as pellets.

8. A composition for use according to any one of claims 1 to 7 wherein the composition is an oral nutritional support.

9. A composition for use according to any one of claims 1 to 8 wherein the composition is a heat treated liquid.

10. A composition for use according to any one of claims 1 to 9 wherein the composition is a liquid meal replacement.

11. A composition for use according to claim 10 wherein the liquid meal replacement is in a form suitable for enteral tube feeding.

12. A composition for use according to any one of claims 1 to 11 wherein the total content of whey protein micelles in the composition is at least 5 wt.%.

13. Non-therapeutic use of a composition comprising complexes of whey protein micelles and pectin to increase satiety and/or postprandial energy expenditure in a subject, wherein the weight ratio of whey protein micelles to pectin in the composition is between 30:1 and 0.8:1, and the whey protein micelles are obtainable by adjusting the pH of a demineralized native whey protein aqueous solution to a value between 5.8 and 6.6 and subjecting the aqueous solution to a temperature between 80 and 98°C for a period of between 10 seconds and 2 hours.

14. A non-therapeutic use according to claim 13 to enhance lean body mass and/or decrease body fat mass.

15. A non-therapeutic use according to claim 13 or claim 14 to help maintain a healthy body composition after weight loss.

## Patentansprüche

1. Zusammensetzung, umfassend Komplexe von Molkenproteinmizellen und Pektin zur Verwendung bei der Behandlung oder Vorbeugung von Fettleibigkeit, wobei das Gewichtsverhältnis von Molkenproteinmizellen zu Pektin in der Zusammensetzung zwischen 30:1 und 0,8:1 liegt und die Molkenproteinmizellen durch Einstellen des pH-Werts einer demineralisierten nativen wässrigen Molkenproteinlösung auf einen Wert zwischen 5,8 und 6,6 und Aussetzen der wässrigen Lösung an eine Temperatur zwischen 80 und 98 °C für einen Zeitraum zwischen 10 Sekunden und 2 Stunden herstellbar sind.

2. Zusammensetzung zur Verwendung nach Anspruch 1, wobei die Zusammensetzung in Kombination mit einer Mahlzeit verabreicht wird.

3. Zusammensetzung zur Verwendung nach Anspruch 2, wobei die Mahlzeit Molkenproteinisolate, native oder hydrolysierte Milchproteine, freie Aminosäuren oder eine Kombination davon umfasst.

4. Zusammensetzung zur Verwendung nach einem der Ansprüche 1 bis 3, wobei die Zusammensetzung an ein Kind oder einen erwachsenen Menschen zu verabreichen ist.

5. Zusammensetzung zur Verwendung nach einem der Ansprüche 1 bis 4, wobei die Zusammensetzung an ein Haustier zu verabreichen ist.

6. Zusammensetzung zur Verwendung nach einem der Ansprüche 1 bis 5, wobei die Zusammensetzung in einer Tagesdosis verabreicht wird, um zwischen 0,1 g und 2,0 g Trockengewicht Molkenproteinmizellen und Pektin pro 1 kg Körpergewicht bereitzustellen.

7. Zusammensetzung zur Verwendung nach einem der Ansprüche 1 bis 6, wobei die Zusammensetzung in der Form von einem Getränk, einer Nährstoffzusammensetzung, einem Riegel, Flocken oder als Pellets ist.

8. Zusammensetzung zur Verwendung nach einem der Ansprüche 1 bis 7, wobei die Zusammensetzung eine orale Ernährungshilfe ist.

9. Zusammensetzung zur Verwendung nach einem der Ansprüche 1 bis 8, wobei die Zusammensetzung eine wärmebehandelte Flüssigkeit ist.

10. Zusammensetzung zur Verwendung nach einem der Ansprüche 1 bis 9, wobei die Zusammensetzung ein flüssiger Mahlzeitenersatz ist.

11. Zusammensetzung zur Verwendung nach Anspruch 10, wobei der flüssige Mahlzeitenersatz in einer Form ist, die für enterale Sondenernährung geeignet ist.

12. Zusammensetzung zur Verwendung nach einem der Ansprüche 1 bis 11, wobei der Gesamtgehalt von Molkenproteinmizellen in der Zusammensetzung mindestens 5 Gew.-% beträgt.

13. Nichttherapeutische Verwendung einer Zusammensetzung, die Komplexe von Molkenproteinmizellen und Pektin umfasst, um die Sattheit und/oder den postprandialen Energieverbrauch bei einem Subjekt zu erhöhen, wobei das Gewichtsverhältnis von Molkenproteinmizellen zu Pektin in der Zusammensetzung zwischen 30:1 und 0,8:1 beträgt und die Molkenproteinmizellen durch Einstellen des pH-Werts einer demineralisierten nativen wässrigen Molkenproteinlösung auf einen Wert zwischen 5,8 und 6,6 und Aussetzen der wässrigen Lösung einer Temperatur zwischen 80 und 98 °C für einen Zeitraum zwischen 10 Sekunden und 2 Stunden herstellbar sind.

14. Nichttherapeutische Verwendung nach Anspruch 13, um die Körpermagermasse zu erhöhen und/oder die Körperfettmasse zu verringern.

15. Nichttherapeutische Verwendung nach Anspruch 13 oder Anspruch 14, um das Beibehalten einer gesunden Körperzusammensetzung nach Gewichtsverlust zu unterstützen.

## Revendications

1. Composition comprenant des complexes de micelles de protéines de lactosérum et de pectine pour une utilisation dans le traitement ou la prévention de l'obésité, dans laquelle le rapport pondéral des micelles de protéines de lactosérum à la pectine dans la composition est compris entre 30:1 et 0,8:1, et les micelles de protéines de lactosérum peuvent être obtenues en ajustant le pH d'une solution aqueuse déminéralisée de protéines natives de lactosérum à une valeur comprise entre 5,8 et 6,6 et en soumettant la solution aqueuse à une température comprise entre 80 et 98 °C pendant une période comprise entre 10 secondes et 2 heures.

2. Composition pour utilisation selon la revendication 1, dans laquelle la composition est administrée en combinaison avec un repas.

3. Composition pour utilisation selon la revendication 2, dans laquelle le repas comprend des isolats de protéines de lactosérum, des protéines de lait natives ou hydrolysées, des acides aminés libres, ou une combinaison de ceux-ci.

4. Composition pour utilisation selon l'une quelconque des revendications 1 à 3, dans laquelle la composition est destinée à être administrée à un être humain enfant ou adulte.

5. Composition pour utilisation selon l'une quelconque des revendications 1 à 4, dans laquelle la composition est destinée à être administrée à un animal de compagnie.

6. Composition pour utilisation selon l'une quelconque des revendications 1 à 5, dans laquelle la composition est administrée à une dose journalière pour fournir entre 0,1 g et 2,0 g de poids sec de micelles de protéines de lactosérum et de pectine pour 1 kg de poids corporel.

7. Composition pour utilisation selon l'une quelconque des revendications 1 à 6, dans laquelle la composition est sous la forme d'une boisson, d'une composition nutritionnelle, d'une barre, de flocons ou en tant que granulés.

8. Composition pour utilisation selon l'une quelconque des revendications 1 à 7, dans laquelle la composition est un soutien nutritionnel oral.

9. Composition pour utilisation selon l'une quelconque des revendications 1 à 8, dans laquelle la composition est un liquide traité thermiquement.

10. Composition pour utilisation selon l'une quelconque des revendications 1 à 9, dans laquelle la composition est un substitut de repas liquide.

11. Composition pour utilisation selon la revendication 10, dans laquelle le substitut de repas liquide est sous une forme appropriée pour une alimentation par sonde entérale.

12. Composition pour utilisation selon l'une quelconque des revendications 1 à 11, dans laquelle la teneur totale en micelles de protéines de lactosérum dans la composition est d'au moins 5 % en poids.

13. Utilisation non thérapeutique d'une composition comprenant des complexes de micelles de protéines de lactosérum et de pectine pour augmenter la satiété et/ou la dépense énergétique postprandiale chez un sujet, dans laquelle le rapport pondéral des micelles de protéines de lactosérum à la pectine dans la composition est compris entre 30:1 et 0,8:1, et les micelles de protéines de lactosérum peuvent être obtenues en ajustant le pH d'une solution aqueuse déminéralisée de protéines natives de lactosérum à une valeur comprise entre 5,8 et 6,6 et en soumettant la solution aqueuse à une température comprise entre 80 et 98 °C pendant une période comprise entre 10 secondes et 2 heures.

14. Utilisation non thérapeutique selon la revendication 13, pour renforcer la masse corporelle maigre et/ou diminuer la masse graisseuse corporelle.

15. Utilisation non thérapeutique selon la revendication 13 ou la revendication 14, pour aider à maintenir une composition corporelle saine après une perte de poids.
